# EUROPEAN PATENT APPLICATION

(11) **EP 3 295 992 A1**
(43) Date of publication of application: **21.03.2018**
(21) Application number: 16189532.1
(22) Date of filing: 19.09.2016
(51) Int. Cl.: A61N 2/02

(54) **SIGNAL FOR CONTROLLING ION CHANNELS OF CELLS**

(71) Applicant: Prosystem Health Products GmbH, 20097 Hamburg (DE)
(72) Inventor: Stettin, Jürgen, 20097 Hamburg (DE)
(74) Representative: RGTH

(57) **Abstract**

In order to effectively control ion channels of cells a signal (100) is suggested which comprises a first pulse sequence bundle (12a) and a second pulse sequence bundle (12b), each comprising at least two pulse sequences (11a, 11b, 11c, 11d, 11e, 11f), wherein each pulse sequence (11a, 11b, 11c, 11d, 11e, 11f) comprises at least one electromagnetic pulse (10a, 10b, 10c, 10d 10e, 10f). The signal (100) comprises a pulse sequence distance (13a, 13b, 13c) between adjacent pulse sequences (11a, 11b, 11c, 11d, 11e, 11f), wherein the signal (100) comprises a first pair (14a) of adjacent pulse sequences (11a, 11b, 11c, 11d, 11e, 11f) and a second pair of adjacent pulse sequences (11a, 11b, 11c, 11d, 11e, 11f), each comprising two pulse sequences (11a, 11b, 11c, 11d, 11e, 11f), wherein the signal (100) comprises the same pulse sequence distance between the adjacent pulse sequences (11a, 11b) of the first pair (14a) of pulse sequences (11a, 11b, 11c, 11d, 11e, 11f) and between the adjacent pulse sequences (11d, 11e) of the second pair of pulse sequences (11a, 11b, 11c, 11d, 11e, 11f).

## Description

### Field of the invention

The invention relates to a signal for controlling ion channels of cells, a method of producing such a signal as well as a method for controlling ion channels. In a further aspect, the invention relates to an apparatus for producing a signal for controlling ion channels of cells.

### Description of related art

It is known in prior art that cancer cells can be destroyed by inserting calcium ions into the cytoplasm, especially the mitochondria, of the cancer cells. US 9,295,835 B2 for example discloses that electrical signals can induce the insertion of calcium ions into the cytoplasm of cancer cells. However, the exact nature or characteristics of the used signals are not discussed or specified in any way.

### Summary of the invention

It is an objective of the present invention to design a signal for controlling ion channels of cells in such a way that the control, especially the activation or deactivation, of the ion channels is as effective as possible. In particular, the signal should be adapted to the biophysical and electrical properties of the cells to which the ion channels belong.

The above objective is solved by providing a signal for controlling ion channels of cells, wherein the signal comprises a first pulse sequence bundle and a second pulse sequence bundle, each comprising at least two pulse sequences. Every pulse sequence comprises at least one electromagnetic pulse. The signal comprises a first pair of adjacent pulse sequences and a second pair of adjacent pulse sequences, each comprising two pulse sequences. The signal comprises a pulse sequence distance between adjacent pulse sequences, wherein the signal comprises the same pulse sequence distance between the adjacent pulse sequences of the first pair of pulse sequences and between the adjacent pulse sequences of the second pair of pulse sequences.

The signal is especially configured to control voltage gated calcium channels of cells. In this regard, the signal is in particular configured to control T-type calcium channels of cells, advantageously all kinds of T-type calcium channels. The cells of whom the voltage gated ion channels are controlled are preferable cancer cells, muscle cells or neurons which further preferred are of human nature. Advantageously, the signal is configured to control ion channels of cells of a human body. Since the signal inhibits the cancer cell growth or leads to the destruction of the cancer cells, the signal serves to reduce or destroy cancer.

The term "pulse sequence distance" relates to the timewise distance between the pulse sequences meaning the length of the time period lying between the pulse sequences.

The first pair of adjacent pulse sequences and the second pair of adjacent pulse sequences differ from each other. This means, that at least one pulse sequence forming the first pair of adjacent pulse sequences is not one of the pulse sequences which form the second pair. Especially, the first pair and the second pair are located directly next to each other. This means that one of the pulse sequences being part of the first pair is also part of the second pair, so that the two pairs are formed by three pulse sequences which are adjacent and spaced apart by the same pulse sequence distance. The pulse sequences forming a pair are preferably from the same pulse sequence bundle. Alternatively, they can also be from different pulse sequence bundles.

The term "adjacent pulse sequences" refers to the fact that one pulse sequence follows another pulse sequence timewise without any other pulse sequence being located in between the two.

The term "signal" is preferably to be understood as a electromagnetic signal which has a duration, i.e. a certain length in time. The second pulse sequence bundle follows the first pulse sequence bundle timewise.

It has been found that applying the signal of the present invention promotes a very effective control of the ion channels of cells. By means of the signal ion channels can be activated, i.e. that a calcium flow into the cell is initiated, or deactivated, meaning that a calcium flow through the ion channels is restricted as long as the signal is applied. Providing the signal in the form of pulse sequences forming pulse sequence bundles guarantees a very effective stimulation of the ion channels. Another major advantage of the present signal is that it is able to control ion channels of cells, especially in humans, non-invasively due to its electromagnetic nature.

In another preferred embodiment, the first pulse sequence bundle and/or the second pulse sequence bundle comprises at least three pulse sequences. The signal preferably comprises a first pulse sequence distance between adjacent pulse sequences of the first pulse sequence bundle and a second pulse sequence distance between adjacent pulse sequences of the second pulse sequence bundle, wherein the first pulse sequence distance is different from the second pulse sequence distance.

Particularly, the first pulse sequence distance is smaller than the second pulse sequence distance. This takes into account the biomechanical properties of ion channels of cells for activation. While the ion channels first respond very well to the stimulation by the pulses, its response becomes less and less with ongoing signal. This is due to a natural exhaustion of the cell, i.e. the channels, for the activation of the channels. By designing the second pulse sequence distance larger than the first pulse sequence distance the signal is especially adapted to the biophysical and electrical properties of the cells, therefore ensuring that the cell can come back into a less exhausted state so that a very effective control of the ion channels is achieved. The pulse sequence distance can also be referred to as a relaxation phase. This term explicitly refers to the biomechanical effect of the relaxation phase on the cells to be controlled.

For deactivating or closing the ion channels, the pulse sequence distances are smaller with ongoing signal. This means that the first pulse sequence distance is greater than the second pulse sequence distance. Alternatively, the pulse sequence distances begin small and stay this way through ongoing signal. This means that the first pulse sequence distance is the same as the second pulse sequence distance and all following pulse sequence distances.

In particular, adjacent pulse sequences of the first pulse sequence bundle each are spaced apart by the first pulse sequence distance, while furthermore adjacent pulse sequences of the second pulse sequence bundle each have the second pulse sequence distance located between them.

Particularly preferred, the first pulse sequence bundle and/or the second pulse sequence bundle comprises between 3 and 20, preferably between 4 and 15, especially preferred between 5 and 8 pulse sequences. In particular, the first pulse sequence bundle and/or a second pulse sequence bundle comprise exactly six pulse sequences.

Further preferred, the signal comprises a third pulse sequence bundle, wherein the signal comprises a first pulse sequence bundle distance between the first pulse sequence bundle and the second pulse sequence bundle and a second pulse sequence bundle distance between the second pulse sequence bundle and the third pulse sequence bundle, wherein the second pulse sequence bundle distance is greater than the first pulse sequence bundle distance.

The first and/or the second pulse sequence bundle distance corresponds preferably to the pulse sequence distance between the pulse sequences of one of the adjacent pulse sequences bundles. In other words, the first pulse sequence bundle distance corresponds preferably to the pulse sequence distance in the first pulse sequence bundle or the pulse sequence distance in the second pulse sequence bundle, while the second pulse sequence bundle distance corresponds to the pulse sequence distance in the second pulse sequence bundle or the third sequence bundle. The pulse sequence bundle distance can be understood as the pulse sequence distance between the last pulse sequence of one pulse sequence bundle and the first pulse sequence of the following pulse sequence bundle.

Further preferred, the signal comprises more than three pulse sequence bundles wherein between each of the pulse sequence bundles there is a pulse sequence bundle distance which is especially designed as explained before.

Advantageously, the first pulse sequence distance and/or the second pulse sequence distance is between 0,5 ms and 500 ms, preferably between 10 ms and 250 ms, especially preferred between 40 ms and 160 ms. In particular, the pulse sequence bundle distance between different pulse sequence bundles increases with the length of the signal, preferably at least within different signal parts. Furthermore, the pulse sequence distance increases with every pulse sequence bundle of the signal, while at the same time it remains constant within the respective pulse sequence bundle.

Wherein a shorter pulse sequence distance is sufficient for a certain relaxation of the cell at the beginning of an application of the signal, the pulse sequence distance has to be increased in time or in other words length of the signal, since at that stage more time is required for the same relaxation effect.

Further preferred, the at least one electromagnetic pulse has a magnetic flux density of between 0,1 mT and 10 mT, preferably between 0,5 mT and 5 mT, especially preferred between 1 mT and 2 mT. The magnetic flux density of the at least one electromagnetic pulse is designed such that it creates an induced voltage strong enough to affect the voltage gated ion channels.

The at least one electromagnetic pulse has preferably a pulse duration between 0,1 ms and 50 ms, preferably between 1 ms and 10 ms, further preferred between 3 ms and 8 ms, especially preferred between 5 ms and 7 ms. Especially, the pulse duration is around 6 ms.

The electromagnetic pulse can be a bipolar or a monopolar pulse. This means, that the pulse has a first part and in case of a bipolar pulse an inverted second part which are adjacent to each other and congruent in shape. In case of a sequence of bipolar pulses, an alternating voltage is supplied to a coil producing the electromagnetic pulses resulting in an overall electromagnetic field whose direction in space alternates. The electromagnetic pulse is in particular a rectangular pulse, a sawtooth pulse, a triangular pulse or a sinus pulse. Furthermore, the electromagnetic pulse can have the shape of a charging or discharging curve of an RC element.
For producing a sequence of monopolar pulses, the voltage supplied to the coil consists of non-alternating voltage peaks which could for example be achieved by a half-wave rectification of the above described alternating voltage used for producing bipolar pulses. Therefore, the electromagnetic field comprises peaks into the same direction distanced by time intervals of no magnetic flux density.

Preferably, the pulse sequences comprise between 1 and 15, preferably between 2 and 10, especially preferred between 3 and 8, electromagnetic pulses. Advantageously, the pulse sequences comprise 6 electromagnetic pulses.

In particular, the pulses forming a pulse sequence are immediately following each other, this means that there is no relevant zero signal between the pulses, except for the zero-crossing due to the inverting process when another pulse begins. Further preferred, the pulses of a pulse sequence are identical to each other, this means that a pulse sequence consists of identical pulses or in other words one electromagnetic pulse which periodically repeats itself. Furthermore, the pulse sequences are preferably identical to each other.

Advantageously, the signal comprises a first signal part and a second signal part, each comprising at least two pulse sequence bundles, wherein the signal comprises a signal part distance between the first signal part and the second signal part.

Especially, the first signal part and the second signal parts each comprise at least two pulse sequence bundles. The signal part distance between the first signal part and the second signal part is preferably referred to as a recovery phase. It takes into account the effect that after a certain signal length the ion channels do not respond to the applied signal anymore, since their response is exhausted. In this case, the relaxation phase is not sufficient anymore to relax the cells so that a good response to the activation or deactivation is achieved. This is why a longer recovery phase has to be applied, so that the response of the ion channels is set to its original state by their recovery.

The recovery phase is to be applied after the relaxation phase reaches a certain duration, wherein the relaxation phase reaches a duration of between 1 ms and 1 s, preferably between 1 ms and 550 ms, especially preferred between 40 ms and 250 ms. Most preferably, the recovery phase is to be applied after the relaxation phase reaches a duration of 250 ms.

Alternatively, the recovery phase is to be applied after a certain amount of signal repetitions, wherein the number of repetitions is between 2 and 100 repetitions, preferably between 2 and 75 repetitions, especially preferred between 6 and 50 repetitions.

Advantageously, the first signal part and the second signal part are identical. This means that the pulses, pulse sequences and pulse sequence bundles determining the first signal part are repeated in exactly the same order and distance in the second signal part. In particular, the signal comprises more than two signal parts, wherein between each adjoining signal parts a signal part distance is located so that the cell can recover from the respective proceeding signal part.

The signal part distance is preferably between 1 ms and 5 s, preferably between 500 ms and 4 s, especially preferred between 1 s and 3 s. In particular, the signal part distance is around 2 s.

In a further aspect, the invention relates to a method for producing a signal as described above wherein the method comprises the steps of producing a first pulse sequence bundle and producing a second pulse sequence bundle in such a way that the signal comprises the same pulse sequence distance between adjacent pulse sequences of a first pair of pulse sequences and between adjacent pulse sequences of a second pair of pulse sequences.

Producing the first pulse sequence bundle and/or the second pulse sequence bundle comprises in particular the steps of producing a first pulse sequence of the first pulse sequence bundle, producing no electromagnetic pulse for a first pulse sequence distance and producing a second pulse sequence of the first pulse sequence bundle. Preferably, the step of producing no electromagnetic pulse for the first pulse sequence distance and producing a third pulse sequence of the first pulse sequence bundle can be included such that the first pulse sequence bundle comprises three pulse sequences. When no electromagnetic pulse is produced, the amplitude of the signal is zero.

As a next step, no electromagnetic pulse for a pulse sequence bundle distance is produced. Then producing the second pulse sequence bundle comprises the steps of producing a first pulse sequence of the second pulse sequence bundle, producing no electromagnetic pulse for a second pulse sequence distance and producing a second pulse sequence of the second pulse sequence bundle. Again the steps of producing no electromagnetic pulse for the second pulse sequence distance and producing a third pulse sequence of the second pulse sequence bundle can follow.

In particular, the method comprises the steps of producing more than two pulse sequence bundles. Furthermore, in particularly, a first signal part is produced including a number of pulse sequence bundles which are produced as described above. After producing no electromagnetic pulse for a signal part distance, the method preferably further comprises producing a second signal part which is advantageously identical to the first signal part. Further preferred, the method comprises the steps of producing more than two signal parts which follow each other timewise and are all identical to each other.

In another aspect, the invention is directed to a method for controlling ion channels of cells using a signal as described above. The invention further relates to a method for reducing or destroying cancer in a human body. Therefore, the method for controlling ion channels of cells serves for treating cancer.

In yet another aspect, the invention is directed to a method for influencing the functionality of muscle cells or neurons by controlling their calcium channels. Therefore, the method also serves for treating migraine and muscle tension among other indispositions.

The enormous advantage is that by using the signal of the invention, the method is very effective while at the same time being absolutely non-invasive. In particular, the method includes applying the signal onto at least a part of a human body especially in a region in which the diseases has been identified.

Furthermore, the invention includes an apparatus for producing a signal as described above comprising a unit for producing electromagnetic pulses. In particular, the unit for producing electromagnetic pulses contains at least one coil.

In another embodiment, an electric field is applied to the target tissue with the aid of two or more electrodes. Therefore, the invention includes an apparatus for producing a signal as described above comprising a unit for producing electric field pulses and two or more electrodes.

### Brief Description of the Drawings

The invention will be described below with reference to the following figures which show in schematic representation:
- Figure 1: a time diagram of a signal for controlling ion channels of cells according to the present invention;
- Figure 2: a time diagram of the first signal part of the signal of figure 1; and
- Figure 3: a time diagram of a part of the first pulse sequence bundle of the signal of figures 1 and 2.

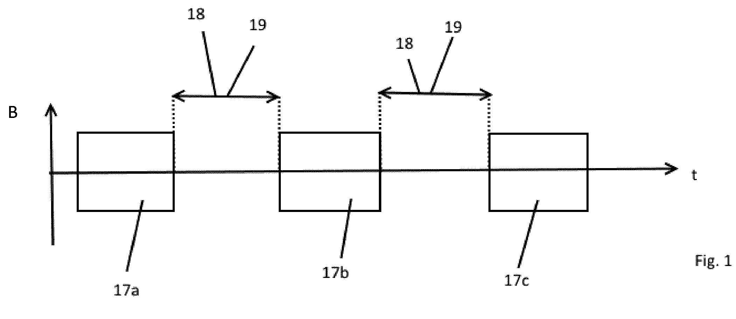

In figure 1 a time diagram of a signal (100) according to the present invention is shown. The time diagram shows the magnetic flux density (B) of the signal (100) versus the time (t). The signal (100) comprises three signal parts (17a, 17b 17c), a first signal part (17a), a second signal part (17b) and a third signal part (17c), wherein between adjacent signal parts a signal part distance (18) is disposed. This means that adjacent signal parts are spaced apart timewise by the signal part distance (18) which is also called recovery phase (19). The different signal parts (17a, 17b, 17c), especially their amplitude of the magnetic flux density (B), are only shown schematically in figure 1. The signal parts (17a, 17b, 17c) are identical to each other. During the recovery phase (19), the amplitude of the signal is zero.

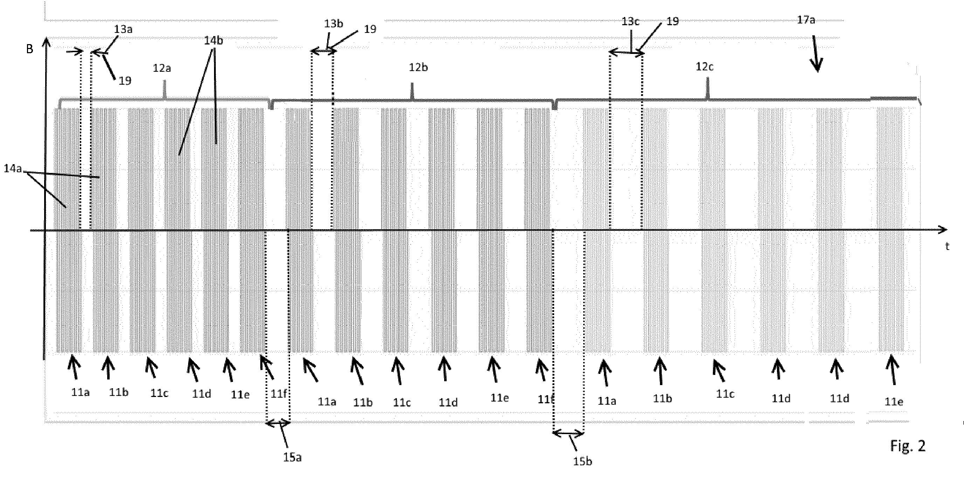

In figure 2 a time diagram of the first signal part (17a) of the signal (100) for controlling ion channels of cells according to figure 1 is shown. The time diagram of figure 1 shows the amplitude of the magnetic flux density (B) of the first signal part (17a) versus the time (t).

The signal comprises three pulse sequence bundles, a first pulse sequence bundle (12a), a following second pulse sequence bundle (12b) and a following third pulse sequence bundle (12c). Each pulse sequence bundle (12a, 12b, 12c) comprises six pulse sequences (11a, 11b, 11c, 11d, 11e, 11f).

The adjacent pulse sequences (11a, 11b, 11c, 11d, 11e, 11f) of the first pulse sequence bundle (12a) are spaced timewise by a first pulse sequence distance (13a). There is a first pair (14a) of pulse sequences, namely of the pulse sequences (11a, 11b), and a second pair (14b) of pulse sequences, namely the pulse sequences (11d, 11e) which each have the first pulse sequence distance (13a) located between their pulse sequences.

In the second pulse sequence bundle (12b) the pulse sequences (11a, 11b, 11c, 11d, 11e, 11f) are spaced apart by a second pulse sequence distance (13b). The adjacent pulse sequences (11a, 11, 11c, 11d, 11e, 11f) of the third pulse sequence bundle (12c) are spaced apart by the third pulse sequence distance (13c).

During the pulse sequence distances (13a, 13b, 13c) the amplitude of the signal is zero. No electromagnetic pulse is produced during these time periods. The pulse sequence distances (13a, 13b, 13c) are also referred to as relaxation phases (19).

Between the pulse sequence bundles (12a, 12b, 12c) the signal (100) comprises different pulse sequence bundle distances. Between the first pulse sequence bundle (12a) and the second pulse sequence bundle (12b) there is the first pulse sequence bundle distance (15a), while between the second pulse sequence bundle (12a) and the third pulse sequence bundle (12c) there is the second pulse sequence bundle distance (15b). The first pulse sequence bundle distance (15a) corresponds to the second pulse sequence distance (13b), whereas the second pulse sequence bundle distance (15b) is equal to the third pulse sequence distance (13c).

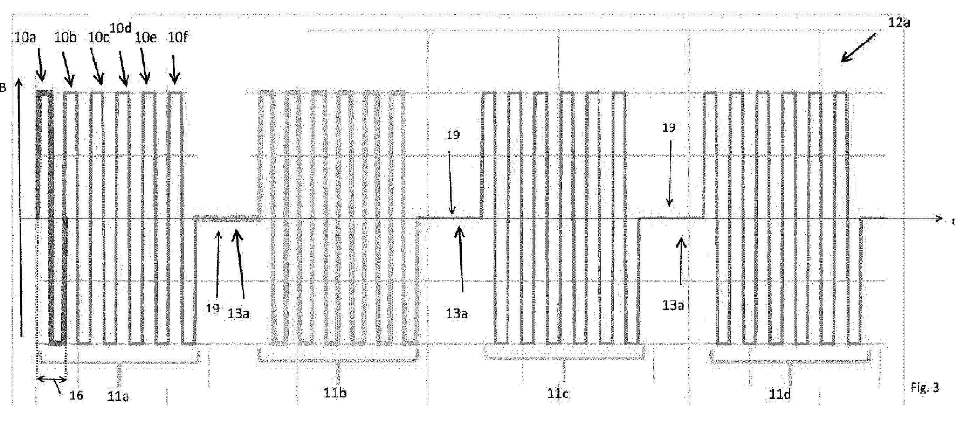

Figure 3 shows a part of the first pulse sequence bundle (12a) of the signal (100) of figures 1 and 2. In detail figure 3 depicts four pulse sequences (11a, 11b, 11c, 11d) of the first pulse sequence bundle (12a) of the first signal part (17a) of the signal (100). Each pulse sequence (11a, 11b, 11c, 11d) comprises six electromagnetic pulses (10a, 10b, 10c, 10d, 10e, 10f) which are identical to each other. The electromagnetic pulses (10a, 10b, 10c, 10d, 10e, 10f) are bipolar rectangular pulses with a pulse duration (16) which follow each other directly within a pulse sequence.

### List of reference signs

- 100: signal
- 10a, 10b, 10c, 10d, 10e, 10f: electromagnetic pulses
- 11a, 11b, 11c, 11d, 11e, 11f: pulse sequences
- 12a: first pulse sequence bundle
- 12b: second pulse sequence bundle
- 12c: third pulse sequence bundle
- 13a: first pulse sequence distance
- 13b: second pulse sequence distance
- 13c: third pulse sequence distance
- 14a: first pair of adjacent pulse sequences
- 14b: second pair of adjacent pulse sequences
- 15a: first pulse sequence bundle distance
- 15b: second pulse sequence bundle distance
- 16: pulse duration
- 17a: first signal part
- 17b: second signal part
- 17c: third signal part
- 18: signal part distance
- 19: relaxation phase
- 20: recovery phase
- B: magnetic flux density
- t: time

## Claims

1. Signal (100) for controlling ion channels of cells,
wherein the signal (100) comprises a first pulse sequence bundle (12a) and a second pulse sequence bundle (12b), each comprising at least two pulse sequences (11a, 11b, 11c, 11d, 11e, 11f),
wherein each pulse sequence (11a, 11b, 11c, 11d, 11e, 11f) comprises at least one electromagnetic pulse (10a, 10b, 10c, 10d 10e, 10f),
wherein the signal (100) comprises a first pair (14a) of adjacent pulse sequences (11a, 11b, 11c, 11d, 11e, 11f) and a second pair (14b) of adjacent pulse sequences (11a, 11b, 11c, 11d, 11e, 11f),
wherein the signal (100) comprises a pulse sequence distance (13a, 13b, 13c) between adjacent pulse sequences (11a, 11b, 11c, 11d, 11e, 11f), **characterized in that**
the signal (100) comprises the same pulse sequence distance between the adjacent pulse sequences (11a, 11b) of the first pair (14a) of pulse sequences (11a, 11b, 11c, 11d, 11e, 11f) and between the adjacent pulse sequences (11d, 11e) of the second pair (14b) of pulse sequences (11a, 11b, 11c, 11d, 11e, 11f).

2. Signal (100) according to claim 1,
wherein the first pulse sequence bundle (12a) and/or the second pulse sequence bundle (12b) comprises at least three pulse sequences (11a, 11b, 11c, 11d, 11e, 11f),
wherein the signal (100) comprises a first pulse sequence distance (13a) between adjacent pulse sequences (11a, 11b, 11c, 11d, 11e, 11f) of the first pulse sequence bundle (12a) and a second pulse sequence distance (13b) between adjacent pulse sequences (11a, 11b, 11c, 11d, 11e, 11f) of the second pulse sequence bundle (12b),
wherein the first pulse sequence distance (13a) is different from the second pulse sequence distance (13b).

3. Signal (100) according to any of claims 1 or 2,
**characterized in that**
the first pulse sequence bundle (12a) and/or the second pulse sequence bundle (12b) comprises between 3 and 20, preferably between 4 and 15, especially preferred between 5 and 8 pulse sequences (11a, 11b, 11c, 11d, 11e, 11f).

4. Signal (100) according to any of the proceeding claims,
**characterized in that**
the signal (100) comprises a third pulse sequence bundle (12c),
wherein the signal (100) comprises a first pulse sequence bundle distance (15a) between the first pulse sequence bundle (12a) and the second pulse sequence bundle (12b) and a second pulse sequence bundle distance (15b) between the second pulse sequence bundle (12b) and the third pulse sequence bundle (12c),
wherein the second pulse sequence bundle distance (15b) is greater than the first pulse sequence bundle distance (15a).

5. Signal (100) according to any of the proceeding claims,
**characterized in that**
the first pulse sequence distance (13a) and/or the second pulse sequence distance (13b) is between 0,5 ms and 500 s, preferably between 10 ms and 250 ms, especially preferred between 40 ms and 160 ms.

6. Signal (100) according to any of the proceeding claims,
**characterized in that**
the at least one electromagnetic pulse (10a, 10b, 10c, 10d, 10e 10f) has a magnetic flux density (B) of between 0,1 mT and 10 mT, preferably between 0,5 mT and 5 mT, especially preferred between 1 mT and 2 mT.

7. Signal (100) according to any of the proceeding claims,
**characterized in that**
the pulse sequences (11a, 11b, 11c, 11d, 11e, 11f) comprise between 1 and 15, preferably between 2 and 10, especially preferred between 3 and 8, electromagnetic pulses (10a, 10b, 10c, 10d, 10e, 10f).

8. Signal (100) according to any of the proceeding claims,
**characterized in that**
the signal (100) comprises a first signal part (17a) and a second signal part (17b), each comprising at least two pulse sequence bundles (12a, 12b 12c), wherein the signal (100) comprises a signal part distance (18) between the first signal part (17a) and the second signal part (17b).

9. Signal (100) according to claim 8,
**characterized in that**
the first signal part (17a) and the second signal part (17) are identical.

10. Signal (100) according to any of claims 8 or 9,
**characterized in that**
the signal part distance (18) is between 1 ms and 5 s, preferably between 500 ms and 4 s, especially preferred between 1 s and 3 s.

11. Method for producing a signal (100) according to any of claims 1 to 9,
**characterized in that**
the method comprises the following steps:
- Producing a first pulse sequence bundle (12a) and
- Producing a second pulse sequence bundle (12b) in such a way that the signal (100) comprises the same pulse sequence distance between the adjacent pulse sequences (11a, 11b) of the first pair (14a) of pulse sequences (11a, 11b, 11c, 11d, 11e, 11f) and between the adjacent pulse sequences (11d, 11e) of the second pair of pulse sequences (11a, 11b, 11c, 11d, 11e, 11f).

12. Method according to claim 11,
**characterized in that**
producing the first pulse sequence bundle (12a) and/or the second pulse sequence bundle (12b) comprises the following steps:
- Producing a first pulse sequence (11a) of the first pulse sequence bundle (12a),
- Producing no electromagnetic pulse (10a, 10b, 10c, 10d 10e, 10f) for a first pulse sequence distance (13a),
- Producing a second pulse sequence (11b) of the first pulse sequence bundle (12a),
- Preferably producing no electromagnetic pulse (10a, 10b, 10c, 10d 10e, 10f) for the first pulse sequence distance (13a) and producing a third pulse sequence (11c) of the first pulse sequence bundle (12a),
- Producing no electromagnetic pulse (10a, 10b, 10c, 10d 10e, 10f) for a pulse sequence bundle distance (15a, 15b),
- Producing a first pulse sequence (11a) of the second pulse sequence bundle (12b),
- Producing no electromagnetic pulse (10a, 10b, 10c, 10d 10e, 10f) for a second pulse sequence distance (13b),
- Producing a second pulse sequence (11b) of the second pulse sequence bundle (12b), and
- Preferably producing no electromagnetic pulse (10a, 10b, 10c, 10d 10e, 10f) for the second pulse sequence distance (13b) and producing a third pulse sequence (11c) of the second pulse sequence bundle (12b).

13. Method for controlling ion channels of cells,
**characterized in that**
the method uses a signal (100) according to any of claims 1 to 9.

14. Apparatus for producing a signal (100) according to any of claims 1 to 9,
**characterized in that**
the apparatus comprises a unit for producing electromagnetic pulses.

15. Apparatus according to claim 14,
**characterized in that**
the unit for producing electromagnetic pulses is a coil, said coil preferably comprising between 50 and 150, further preferred between 70 and 120, especially preferred between 90 to 110, turns.
